# EUROPEAN PATENT APPLICATION

(11) **EP 4 623 802 A1**
(43) Date of publication of application: **01.10.2025**
(21) Application number: 25165277.2
(22) Date of filing: 21.03.2025
(51) Int. Cl.: A61B 3/00

(54) **OPHTHALMOLOGIC APPARATUS**

(30) Priority: 26.03.2024 JP 2024048893
(71) Applicant: TOPCON CORPORATION, Tokyo 174-8580 (JP)
(72) Inventor: ITO, Ryousuke, Tokyo, 174-8580 (JP); MOCHIZUKI, Reina, Tokyo, 174-8580 (JP)
(74) Representative: Louis Pöhlau Lohrentz

(57) **Abstract**

An ophthalmologic apparatus (A, A') includes a measurement portion (20) that is configured to measure a subject eye (E), a cover body (30) that is configured to cover the measurement portion (20), a monitor (50) that is disposed outside of the cover body (30) and is configured to present a touch-panel screen (51) at a monitor position of the monitor (50), and a moving mechanism that is configured to move the monitor position of the monitor (50). The moving mechanism is a link mechanism (70, 70') including at least first and second vertical shafts (73, 73', 75) and at least first and second connecting arms (74, 74', 76). The first and second connecting arms (74, 74', 76) are rotatably connected by the first and second vertical shafts (73, 73', 75) as a joint therebetween. The link mechanism (70, 70') is configured to move the monitor position of the monitor (50) within a predetermined movement range between a front-side position and a back-side position in a front-back axis direction of the ophthalmologic apparatus (A, A') at an upper height position on a side surface of the cover body (30).

## Description

### FIELD OF THE INVENTION

The present invention relates to an ophthalmologic apparatus.

### BACKGROUND

It is known in the art that an ophthalmologic apparatus includes a moving mechanism that adjusts the monitor position (or display position) of a monitor (or display). JP2018-000646A discloses an ophthalmologic apparatus for examining subject eyes which includes a moving mechanism. This moving mechanism includes an eye examination portion that examines subject eyes, a movable portion that moves a display portion in a horizontal direction relative to the eye examination portion, and a guide portion that guides the movement of the movable portion.

Two types of ophthalmologic apparatus are known in the art: an ophthalmologic apparatus with a lever (i.e., lever-equipped ophthalmologic apparatus) and an ophthalmologic apparatus without a lever (i.e., lever-free ophthalmologic apparatus). The lever-equipped ophthalmologic apparatus includes a control lever for controlling the movement of the measurement portion relative to the apparatus pedestal in three axis directions. The lever-free ophthalmologic apparatus uses a touch panel operation on the screen of the monitor without a control lever. A monitor position that ensures high operability is required, regardless of whether the examiner selects the lever or touch-panel operation when operating from the side in the lever-equipped ophthalmologic apparatus. Furthermore, a common moving mechanism for adjusting the monitor position is required, regardless of whether the ophthalmologic apparatus is lever-equipped or lever-free, to avoid the need for separate mechanisms for the lever-equipped and lever-free ophthalmologic apparatuses. The lever operation may include a combination of the control lever operation and the touch-panel operation.

The moving mechanism disclosed in JP2018-000646A is a sliding guide mechanism that includes rollers mounted on an attachment plate (movable portion) and a guide groove (guide portion) in a base portion. The sliding guide mechanism adjusts the position of the monitor by engaging the rollers with the guide groove and allowing movement along it. This moving mechanism needs to allow the monitor to move along the side surface of the cover body in a predetermined range in the front-back axis direction when the examiner seeks to achieve both lever operability and touch-panel operability while operating from the side. This presents a challenge in which the sliding guide mechanism must be configured with a long, bent guide groove in a large base portion and a plurality of rollers to ensure stable engagement with the groove, leading to higher costs due to increased processing costs and a greater number of components.

A conventional moving mechanism used in ophthalmologic apparatuses is a so-called arm mechanism, which includes a single vertical shaft provided at the center of the top surface of the cover body and a single arm extending horizontally from the vertical shaft as a pivot axis. This arm mechanism moves the monitor along a semicircular path centered on the single vertical shaft, with the arm length as its radius, and allows positioning only in the tangential direction of this semicircular path. In other words, this arm mechanism has limited flexibility in setting the monitor position and does not allow positioning at different locations along the front-back axis on the side surface of the cover body. Therefore, this arm mechanism does not allow switching the monitor position to accommodate both the lever operation and the touch-panel operation when the examiner operates from the side.

The present invention addresses the above issue. An object of the present invention is to provide an ophthalmologic apparatus with an economically advantageous moving mechanism that enables a monitor position to be switched according to a selected operation target when an operator such as an examiner operates from a side of the ophthalmologic apparatus.

### SUMMARY

According to one embodiment of the present invention, an ophthalmologic apparatus includes a measurement portion that is configured to measure a subject eye; a cover body that is configured to cover the measurement portion; a monitor that is disposed outside of the cover body and is configured to present a touch-panel screen at a monitor position of the monitor; and a moving mechanism that is configured to move the monitor position of the monitor. The moving mechanism is a link mechanism. The link mechanism includes at least first and second vertical shafts and at least first and second connecting arms. The first and second connecting arms are rotatably connected by the first and second vertical shafts as a joint therebetween. The link mechanism is configured to move the monitor position of the monitor within a predetermined movement range between a front-side position and a back-side position in a front-back axis direction of the ophthalmologic apparatus at an upper height position on a side surface of the cover body.

The ophthalmologic apparatus of the present invention uses the link mechanism with at least two vertical shafts as the moving mechanism, providing an economically advantageous solution while enabling the monitor position to be switched according to the selected operation target when the examiner operates from the side.

### BRIEF DESCRIPTION OF DRAWINGS

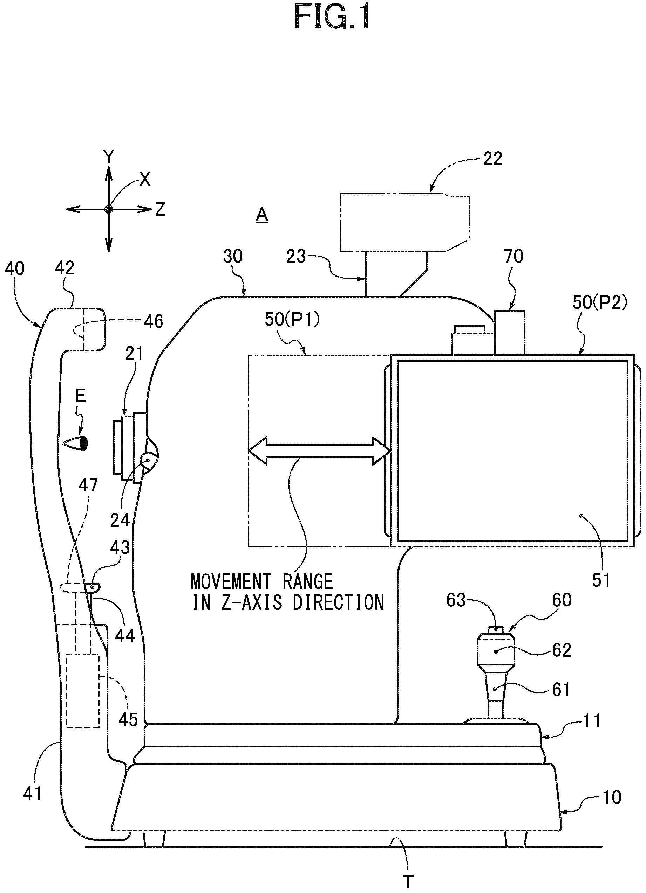

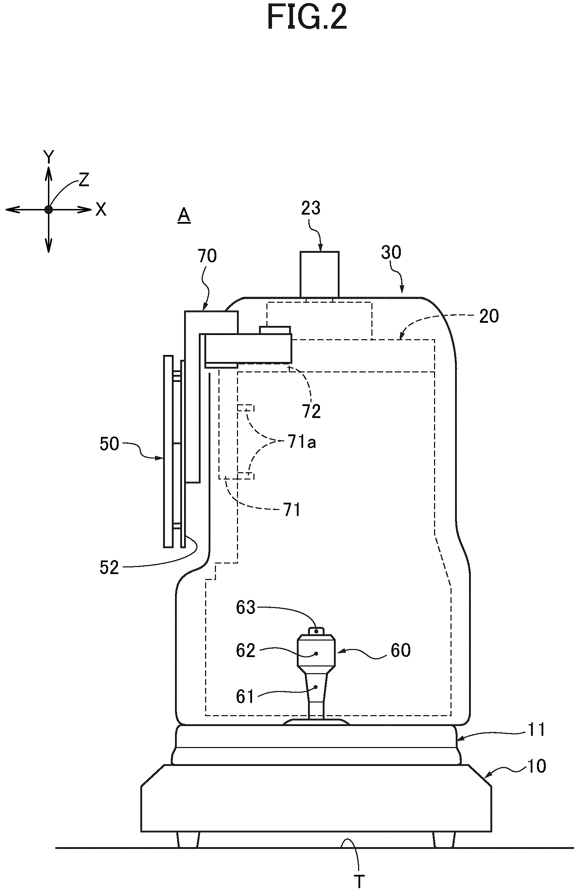

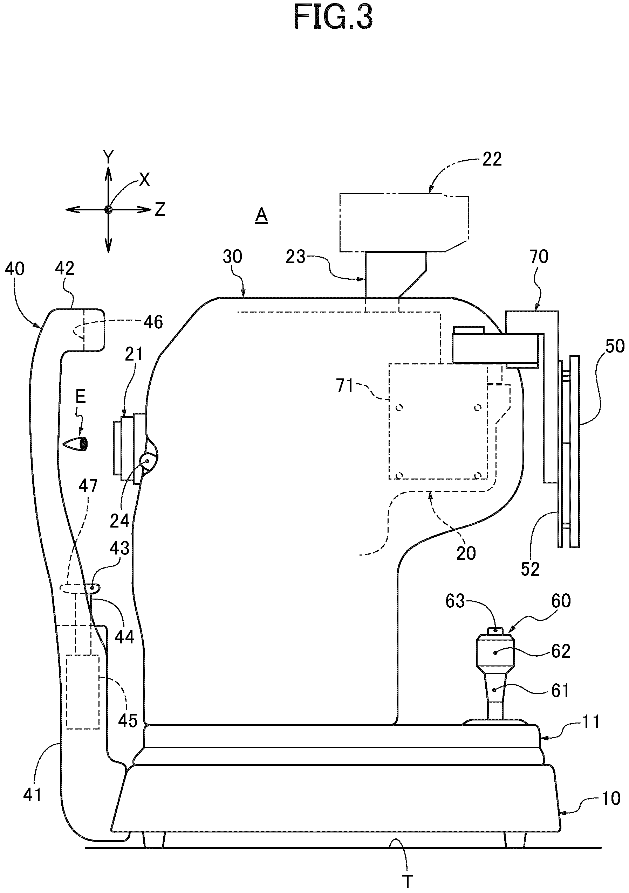

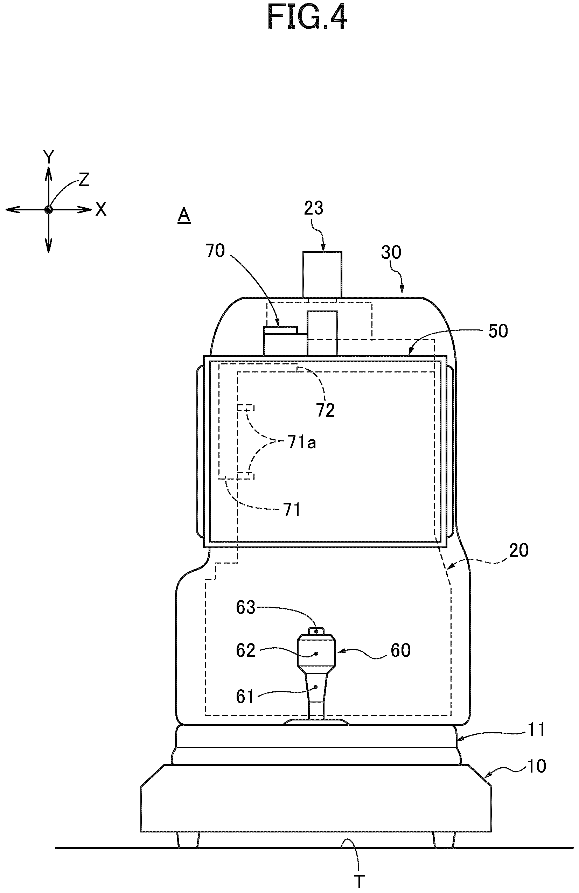

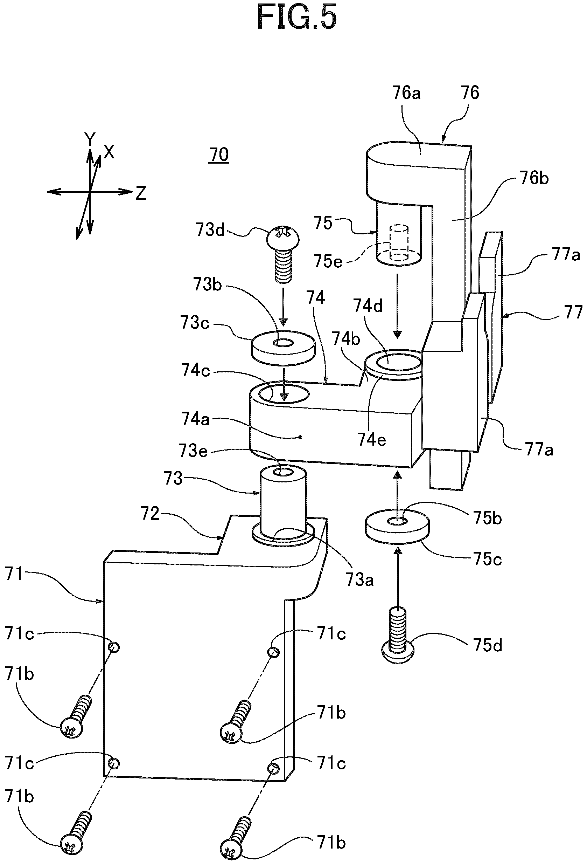

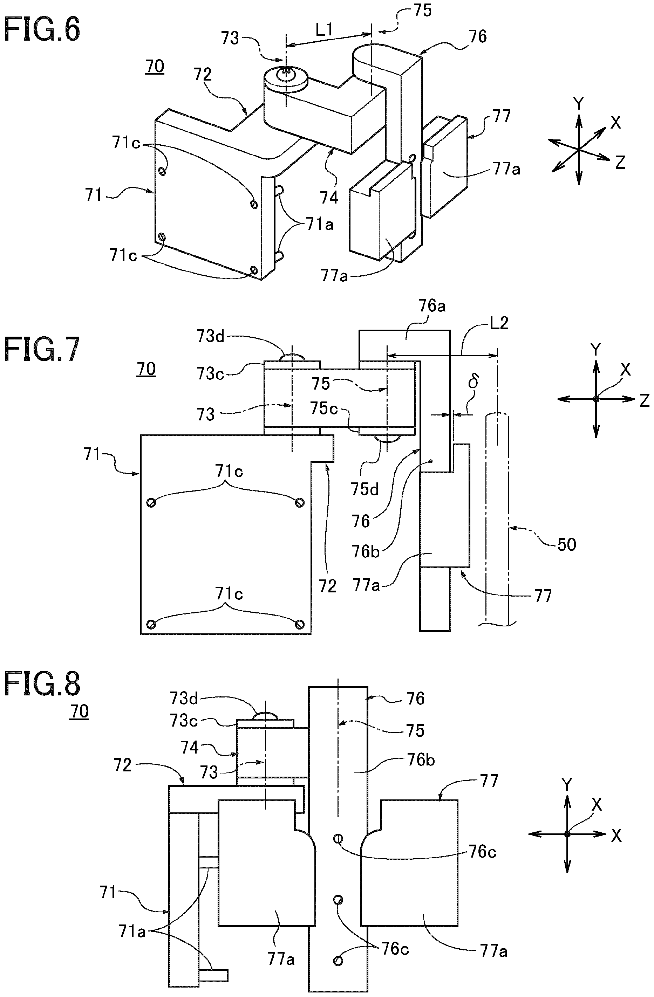

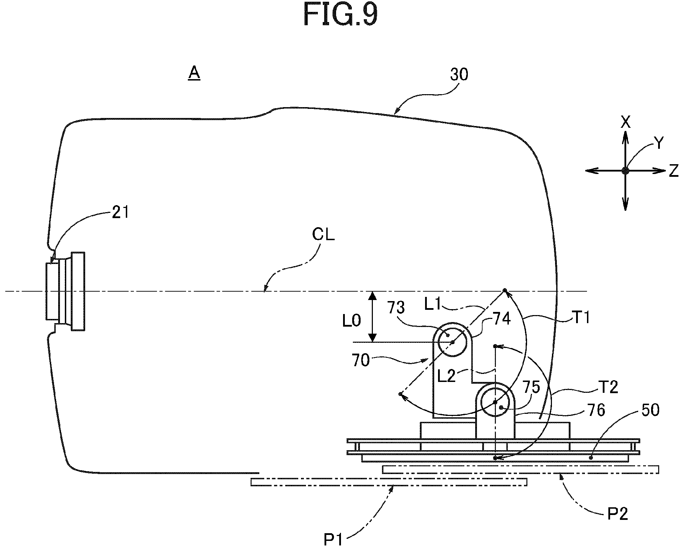

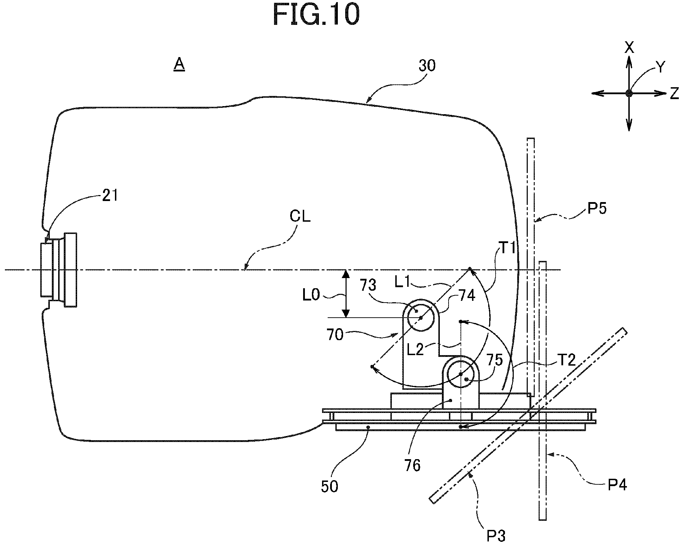

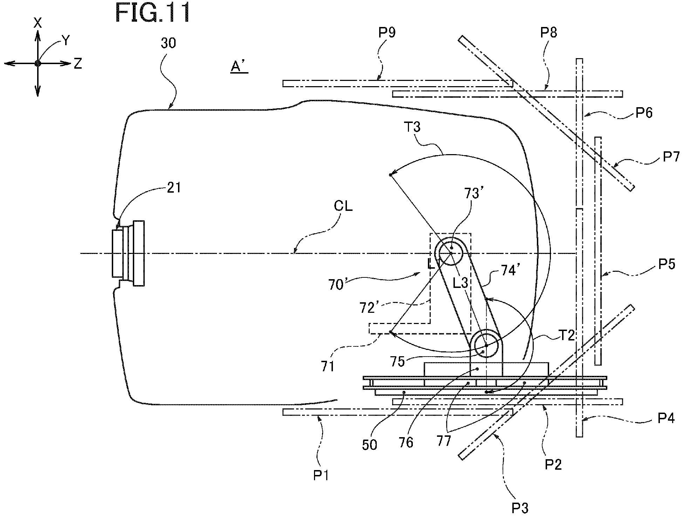

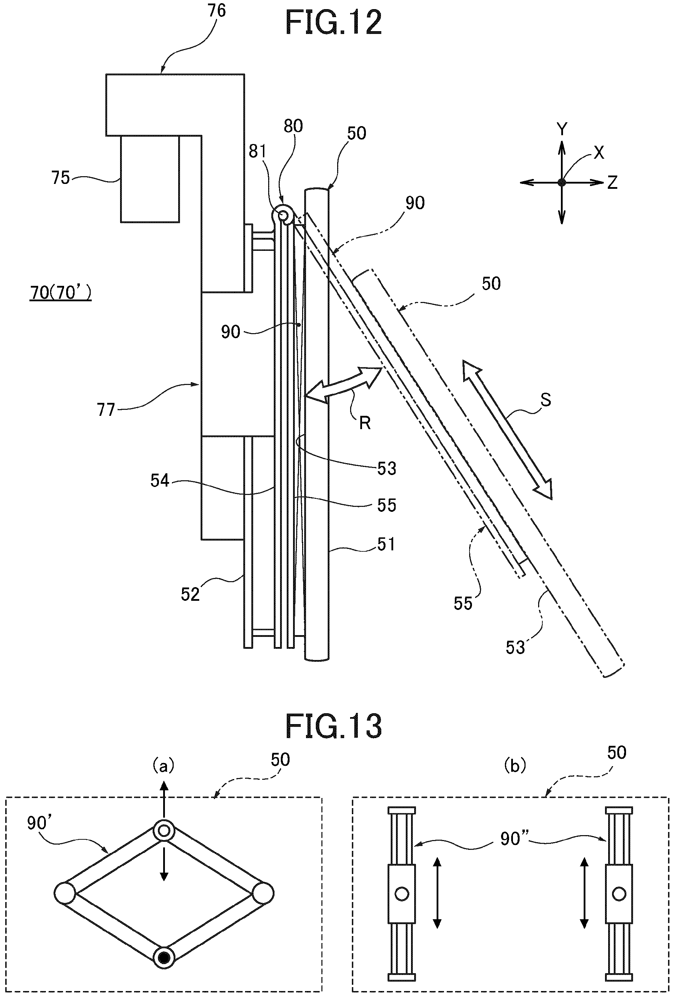

FIG. 1 is a side view of an ophthalmologic apparatus according to a first embodiment, with the monitor set on its side surface. FIG. 2 is a rear view of the ophthalmologic apparatus according to the first embodiment, with the monitor set on its side surface. FIG. 3 is a side view of the ophthalmologic apparatus according to the first embodiment, with the monitor set on its back surface. FIG. 4 is a rear view of the ophthalmologic apparatus according to the first embodiment, with the monitor set on its back surface. FIG. 5 is an exploded perspective view showing a link mechanism according to the first embodiment. FIG. 6 is an assembled perspective view showing the link mechanism according to the first embodiment. FIG. 7 is a side view showing the link mechanism according to the first embodiment. FIG. 8 is a rear view showing the link mechanism according to the first embodiment. FIG. 9 is a plan view showing the setting of the monitor position on the side surface of the cover body. FIG. 10 is a plan view showing the setting of the monitor position on the back surface of the cover body. FIG. 11 is a plan view showing a link mechanism and the setting of the monitor position according to a second embodiment. FIG. 12 is a side view showing a monitor tilting mechanism and a monitor sliding mechanism according to a third embodiment. FIG. 13 is a schematic view showing an example of the monitor sliding mechanism according to the third embodiment.

### DETAILED DESCRIPTION

With respect to the use of plural and/or singular terms herein, those having skill in the art can translate from the plural to the singular and/or from the singular to the plural as is appropriate to the context and/or application. The various singular/plural permutations may be expressly set forth herein for the sake of clarity.

A configuration for implementing an ophthalmologic apparatus according to the present invention will be described, based on first to third embodiments shown in the drawings.

The ophthalmologic apparatus, which is applied to the first to third embodiments, observes, captures, and records ocular fundus images of the subject eyes and provides them as electronic images for diagnosis. With the examinee facing a body of the ophthalmologic apparatus, the X, Y, and Z in the drawings respectively indicate the X-axis as the left-right axis in the left-right direction (horizontal direction), the Y-axis as the vertical axis in the up-down direction (vertical direction), and the Z-axis as the front-back axis (depth direction) orthogonal to the X and Y axes.

### First Embodiment

As shown in FIGS. 1 to 4, an ophthalmologic apparatus A includes an apparatus pedestal 10, a measurement portion 20, a cover body 30, a face support 40, a monitor 50, a control lever 60, and a link mechanism (moving mechanism) 70.

The apparatus pedestal 10 is a base member to be placed, for example, on an optical table T. The optical table T is height-adjustable in the Y-axis direction. The apparatus pedestal 10 includes an XZ movable frame 11 mounted on it. The measurement portion 20 is mounted on the XZ movable frame 11 and covered with the cover body 30. The face support 40 is fixed to the front side of the apparatus pedestal 10, where the subject eyes E are positioned.

The measurement portion 20 includes an optical system in an optical head frame (not shown) and is configured to observe and capture the ocular fundus images of the subject eyes E. The optical head frame is movable in the X-axis, Y-axis and Z-axis directions relative to the apparatus pedestal 10. The optical head frame includes an observation optical system, an illumination optical system, and an alignment optical system as components of the optical system. In the observation optical system and the illumination optical system, an objective lens 21 is positioned at the front, facing the subject eyes E. The observation optical system includes a built-in image sensor that acquires ocular fundus images of the subject eyes E. When capturing ocular fundus images using an external camera 22, the observation optical system includes a camera attachment portion 23 for mounting the camera 22. The camera 22 may be an infrared camera (IR camera) that utilizes infrared light or a visible camera capable of capturing visible light. The alignment optical system includes left and right anterior ocular segment stereo cameras 24 to obtain three-dimensional position information of the pupil of the subject eye E when aligning with the pupil of the subject eye E before observing and capturing an ocular fundus image. The measurement portion 20 is entirely covered with the cover body 30.

The face support 40 is disposed on the front side of the apparatus pedestal 10. The face support 40 is configured to receive and support the forehead and chin of the examinee's face to stabilize the position and orientation of the subject eyes E when observing and capturing an ocular fundus image. The face support 40 includes a chin rest support 41, a forehead rest frame 42, a chin rest base 43, a lifting rod 44, and a driver 45. The chin rest support 41 is fixed to a front end surface of the apparatus pedestal 10. The forehead rest frame 42 is fixed to the chin rest support 41 and includes a forehead rest surface 46. The chin rest base 43 is fixed to the upper end of the lifting rod 44 and includes a chin rest surface 47. The driver 45 is built into the chin rest support 41 and moves the lifting rod 44 in the Y-axis direction during the alignment to adjust the height of the subject eye E.

The monitor 50 is attached to the distal end of the link mechanism 70 and is positioned at an upper position on the outer periphery of the cover body 30. The monitor 50 includes a touch-panel screen 51, which is larger than those used in conventional ophthalmologic apparatuses. The examiner can perform touch-panel operations by touching images displayed on the touch-panel screen 51, such as button images, anterior ocular segment images, and ocular fundus images, with a finger, serving as an input to the controller (not shown).

The control lever 60 is positioned at a lower location on the back side of the cover body 30. The examiner operates the control lever 60, which serves as an input to the controller. The controller electronically controls various components of the ophthalmologic apparatus, including the measurement portion 20 and the face support 40, based on either the lever operation of the control lever 60 or the touch-panel operation on the touch-panel screen 51 of the monitor 50.

The control lever 60 includes a lever portion 61, a ring portion 62, and a shutter button 63. The lever portion 61 extends upright in the Y-axis direction from the top surface of the XZ movable frame 11 and allows tilting operations in the left-right and front-back directions. Tilting the lever in the left-right direction moves the measurement portion 20 relative to the apparatus pedestal 10 in the lever tilting direction along the X-axis. Tilting the lever in the front-back direction moves the measurement portion 20 relative to the apparatus pedestal 10 in the lever tilting direction along the Z-axis. The ring portion 62 is positioned on the upper outer circumference of the lever portion 61 and allows right and left rotational operations. Rotating the ring to the right moves the measurement portion 20 upward along the Y-axis relative to the XZ movable frame 11. Rotating the ring to the left moves the measurement portion 20 downward along the Y-axis relative to the XZ movable frame 11. The shutter button 63 is disposed on the top surface of the lever portion 61 and is configured to be pressed by an operator, such as an examiner.

The link mechanism 70 is a moving mechanism that moves or adjusts the monitor position where the touch-panel screen 51 of the monitor 50 is presented to the examiner. As shown in FIGS. 2 to 4, the link mechanism 70 is fixed to an upper position of the measurement portion 20 and exposes most of its structure, including the monitor 50, to the outside of the cover body 30. The link mechanism 70 is configured to move or adjust the monitor position of the monitor 50 within a first predetermined range at the upper height position on the right side of the cover body 30. The first predetermined range includes movement in the Z-axis direction between a front-side position P1 (position shown with the imaginary line in FIG. 1) and a back-side position P2 (position shown with the solid line in FIG. 1). Furthermore, the link mechanism 70 is configured to move or adjust the monitor position of the monitor 50 within a second predetermined range at the upper height position on the back side of the cover body 30. The second predetermined range includes movement in the X-axis direction among a back-right position, a corner inclined position, and a back-center position (position shown with the solid line position). The detailed configuration of the link mechanism 70 will be described later.

Next, the configuration of the link mechanism 70 will be described in detail with reference to FIGS. 5 to 8. The link mechanism 70 functions as a moving mechanism that moves or adjusts the monitor position where the touch-panel screen 51 of the monitor 50 is presented to the examiner. The link mechanism 70 includes at least two vertical shafts and at least two arms, with the vertical shafts serving as joints between them. In this embodiment, the link mechanism 70 is configured with two vertical shafts and two arms and includes a fixation plate 71, a support plate 72, a first vertical shaft 73, a first bent connecting arm (first connecting arm) 74, a second vertical shaft 75, a second connecting arm 76, and a monitor attachment portion 77. The link mechanism 70 is formed, for example, by resin molding using a synthetic resin material. The material of the link mechanism 70 is not limited to synthetic resin but may also be metal or a composite material of metal and synthetic resin.

The fixation plate 71 is fixed to the right-side surface position of the measurement portion 20, when viewed from the front side and to the left-side surface position of the measurement portion 20, when viewed from the back side (see FIGS. 2 and 4). The fixation plate 71 is a rectangular plate and includes four positioning pins 71a extending in the X-axis direction from the right-side surface of the measurement portion 20, and four screw holes 71c through which four screws 71b are inserted. The fixation plate 71 is positioned and fixed to the right-side surface of the measurement portion 20 by fastening the four screws 71b (see FIG. 5).

The support plate 72 is integrally connected to the fixation plate 71 and extends from the upper back side of the fixation plate 71 in the X-axis direction orthogonal to a vertical plate surface of the fixation plate 71. The support plate 72 is placed on the upper surface position of the measurement portion 20 (see FIGS. 2 and 4). The inner attachment surface of the support plate 72 is orthogonal to the attachment surface of the fixation plate 71 so that they are respectively conform to the top surface and the right-side surface of the measurement portion 20. The support plate 72 is positioned and fixed to the top surface of the measurement portion 20 when the fixation plate 71, which is integrally formed with the support plate 72, is positioned and secured to the right-side surface of the measurement portion 20 with fastening screws 71b.

The first vertical shaft 73 is integrally formed at an end position on the top surface of the support plate 72 and extends upward therefrom. The first vertical shaft 73 has a cylindrical shape. The first vertical shaft 73 includes a stepped receiving surface 73a at its base, which allows the first bent connecting arm 74 to contact it with a limited surface area. In a plan view of the cover body 30 (see FIGS. 9 and 10), the first vertical shaft 73 is positioned at an offset position L0. The offset position L0 is located on the rear side of the centerline CL extending in the Z-axis direction (front-back direction) and is offset to the right along the X-axis (left-right axis) from the centerline CL. The first bent connecting arm 74 is connected to the first vertical shaft 73 by inserting the first vertical shaft 73 into a first vertical shaft hole 74c of the first bent connecting arm 74, then placing a first washer 73c with a screw hole 73b on the top surface of the first vertical shaft 73 and fastening them with a first screw 73d. The first washer 73c is fixed onto the top surface of the first vertical shaft 73 by fastening the first screw 73d into a screw hole 73e of the first vertical shaft 73 (see FIG. 5).

The first bent connecting arm 74 includes a radial arm portion 74a and a circumferential arm portion 74b to be bent into an L-shape. The first bent connecting arm 74 is rotatably connected at one end to the first vertical shaft 73. The first bent connecting arm 74 is bent in a direction bringing the second vertical shaft 75 closer to the centerline CL when the first bent connecting arm 74 rotates. The first bent connecting arm 74 includes a first vertical shaft hole 74c and a second vertical shaft hole 74d. The first vertical shaft hole 74c rotatably receives the first vertical shaft 73 and the second vertical shaft hole 74d rotatably receives the second vertical shaft 75. The second vertical shaft hole 74d includes a stepped receiving surface 74e that protrudes from the surrounding top surface, allowing the second connecting arm 76 to contact the stepped receiving surface 74e over a limited surface area. The first bent connecting arm 74 has an arm length L1 between the axis of the first vertical shaft 73 and the axis of the second vertical shaft 75. The arm length L1 is set to allow the monitor 50 to pass over the corner portion between the right-side surface and the back surface of the cover body 30 (see FIGS. 10 and 11). The length of the circumferential arm portion 74b from the bent portion is determined based on the offset distance of the first vertical shaft 73 from the centerline CL and is set to be equal to or slightly greater than the offset distance.

The second vertical shaft 75 is rotatably inserted into the second vertical shaft hole 74d provided at a distal end portion of the circumferential arm portion 74b of the first bent connecting arm 74. The second vertical shaft 75 is integrally formed with the second connecting arm 76 and extends downward from the bottom surface of the second connecting arm 76. The second vertical shaft 75 has a cylindrical shape. The second vertical shaft 75 is rotatably connected with the first bent connecting arm 74 by inserting the second vertical shaft 75 into the second vertical shaft hole 74d, then placing a second washer 75c including a screw hole 75b on the bottom surface of the second vertical shaft 75 and then securing them with a second screw 75d. The second washer 75c is fixed onto the bottom surface of the second vertical shaft 75 by fastening the second screw 75d into a screw hole 75e of the second vertical shaft 75 (see FIG.5).

The second connecting arm 76 includes a horizontal arm portion 76a and a vertical pillar portion 76b. The horizontal arm portion 76a includes the second vertical shaft 75 at a first end thereof and the vertical pillar portion 76b extends downward from a second end of the horizontal arm portion 76a. The vertical pillar portion 76b includes three screw holes 76c for fastening a monitor attachment plate 52 (see FIGS. 2 and 3) fixed to the back surface of the monitor 50 or for adjusting the fastening position as necessary (see FIG. 8). As shown in FIG. 7, the second connecting arm 76 has an arm length L2, which is the distance from the axis of the second vertical shaft 75 to the attachment position of the monitor 50 and is set to be slightly shorter than the arm length L1.

The monitor attachment portion 77 is integrally connected to the vertical pillar portion 76b extending from the second end of the horizontal arm portion 76a, and the back surface of the monitor 50 is attached to the monitor attachment portion 77. The monitor attachment portion 77 includes a pair of clamping portions 77a that are integrally formed with both side surfaces of the vertical pillar portion 76b. The clamping portions 77a are disposed on both sides of the vertical pillar portion 76b and are spaced apart from the front surface of the vertical pillar portion 76b by a predetermined gap δ. The monitor 50 is attached to the monitor attachment portion 77 by inserting the monitor attachment plate 52, which is on the back surface of the monitor 50, into the gap δ between the front surface of the vertical pillar portion 76b and the pair of clamping portions 77a, and then fastening it with the screws (see FIGS. 2 and 3).

Next, the monitor position setting operation onto the cover side surface will be described with reference to FIGS. 1, 2, and 9. The conventional ophthalmologic apparatuses include the lever-equipped ophthalmologic apparatuses that include control levers and the lever-free ophthalmologic apparatuses in which the examiner operates a touch panel on the screen of the monitor, as mentioned above. A monitor position that ensures high operability is required, regardless of whether the examiner selects the lever or touch-panel operation when operating from the side in the lever-equipped ophthalmologic apparatus. Furthermore, a common moving mechanism for adjusting the monitor position is required, regardless of whether the ophthalmologic apparatus is lever-equipped or lever-free, to avoid the need for separate mechanisms for the lever-equipped and lever-free ophthalmologic apparatuses.

As mentioned above, the moving mechanism disclosed in JP2018-000646A is a sliding guide mechanism in which rollers engage with a guide groove. This mechanism increases the cost when configuring it to ensure both lever operability and touch-panel operability for the examiner operating from the side. Another conventional moving mechanism is an arm mechanism that includes a single vertical shaft and a single arm extending in a horizontal direction from the vertical shaft as a pivot axis. Thus, while the arm mechanism can reduce costs compared to the sliding guide mechanism, it cannot fulfill the above-mentioned requirements when the examiner operates from the side.

The present inventors focused on the fact that a link mechanism with at least two vertical shafts can achieve both cost reduction, which is an advantage of the arm mechanism, and a high degree of freedom in setting the monitor position, which is an advantage of the sliding guide mechanism. The ophthalmologic apparatus A includes the measurement portion 20 configured to measure a subject eye E, the cover body 30 that is configured to cover the measurement portion 20, the monitor 50 disposed outside the cover body 30, and a moving mechanism configured to move the monitor position of the monitor 50 where the touch-panel screen 51 of the monitor 50 is presented. The moving mechanism includes a link mechanism 70 including at least two vertical shafts and at least two connecting arms with the connecting arms being connected via the vertical shafts as a joint therebetween. The link mechanism 70 is configured to move or adjust the monitor position of the monitor 50 within a first predetermined range at the upper height position on the right side of the cover body 30. The first predetermined range includes movement in the Z-axis direction between the front-side position and the back-side position.

The first bent connecting arm 74 rotates with the first vertical shaft 73 acting as the center and the arm length L1 of the first bent connecting arm 74 acting as the radius, causing the second vertical shaft 75 of the link mechanism 70 to move along a movement trajectory T1, forming a semicircular path within a first angle range. For example, the first angle range is 180 degrees or approximately 180, but is not limited to these values. Then, the second connecting arm 76 rotates with the second vertical shaft 75 acting as the center and the arm length L2 serving as the radius, causing the monitor 50, which is attached to the distal end of the link mechanism 70, to move along a movement trajectory T2, forming a semicircular path within a second angle range. For example, the second angle range is 180 degrees or approximately 180, but is not limited to these values. Thus, the total movement range of the monitor position is determined by the combination of the movement trajectory T1 and the movement trajectory T2. Accordingly, when the movement trajectory T1 is within a predetermined angle range on the lower side, which is shown in FIG. 9, of the entire movement trajectory T1, it defines a movement range that substantially follows the right-side surface of the cover body 30. For example, the predetermined angle range on the lower side is 90 degrees or approximately 90 but is not limited to these values. Once the position of the second vertical shaft 75 is fixed in the movement trajectory T1, the angle of the touch-panel screen 51 of the monitor 50 can be adjusted within the movement trajectory T2 so that it is positioned parallel to the side surface of the cover body 30.

The link mechanism 70 is configured to move the monitor position of the monitor 50 within a movement range in the Z-axis direction between the front-side position P1 and the back-side position P2 at an upper height position on the right-side surface of the cover body 30, as shown in FIG. 9. When the touch-panel screen 51 of the monitor 50 is set at the back-side position P2 at an upper height position on the right-side surface of the cover body 30, as shown with the solid lines in FIGS. 1 and 2, the examiner's position becomes closer to the control lever 60, making it more suitable for the lever operation from the side. In contrast, when the touch-panel screen 51 of the monitor 50 is set at the front-side position P1 at an upper height position on the right-side surface of the cover body 30, the examiner's position becomes closer to the examinee, making it more suitable for the touch-panel operation from the side.

The ophthalmologic apparatus A includes the above-explained link mechanism 70 including the first and second vertical shafts 73, 75 as the moving mechanism to move the monitor position of the monitor 50. Therefore, the ophthalmologic apparatus A can selectively set the monitor position to correspond to the selection of the operation target when the examiner operates from the side position. As a result, the ophthalmologic apparatus A can switch the monitor position between the lever operation and the touch-panel operation from the side. Furthermore, the link mechanism 70 has the flexibility to accommodate both the lever operation and the touch-panel operation, and accordingly, the same link mechanism 70 can be commonly used for both the lever-equipped ophthalmologic apparatuses and the lever-free ophthalmologic apparatuses.

The monitor position setting operation onto the cover back surface will be described with reference to FIGS. 3, 4, and 10. When the examiner sits at the back side of the cover body 30 and performs either the touch-panel operation or the lever operation, or both, the examiner prefers to set the monitor 50 at a central position on the upper back surface of the cover body 30, near the second vertical shaft 75, to ensure high operability for both operations.

When the link mechanism 70 is installed on the measurement portion 20 as in the first embodiment, the first vertical shaft 73 may need to be positioned at the offset position L0, which is offset from the center line CL, due to positional constraints imposed by an ocular fundus camera or other components attached to the measurement portion 20. At this time, if the link mechanism 70 uses a second connecting link that is a straight link, positional constraints imposed by the measurement portion 20, similar to those affecting the first vertical shaft 73, may prevent the second vertical shaft 75 from rotating into a position on the center line CL. If the second vertical shaft 75 cannot be positioned on the center line CL, the monitor 50 may not be able to be set at a central position on the upper back surface of the cover body 30, making it difficult to ensure high operability for both operations.

In contrast, the first connecting arm according to the first embodiment is formed into the first bent connecting arm 74 that is bent at a bent portion to be an L-shape with a radial arm portion 74a and a circumferential arm portion 74b. The circumferential arm portion 74b extends from the bent portion toward the back surface of the cover body 30, while the radial arm portion 74a extends toward the right-side surface of the cover body 30 from the first vertical shaft 73 offset from the center line CL.

As mentioned above, the angle range to move the monitor position of the monitor 50 is determined by the combination of the movement trajectory T1 and the movement trajectory T2. Accordingly, when the movement trajectory T1 is within a predetermined angle range on the right side, which is shown in FIG. 10, of the entire movement trajectory T1 in FIG. 10, which corresponds to the area moving along the back surface of the cover body 30, it defines a movement range for the movement along the back surface of the cover body 30. For example, the predetermined angle range on the right side is 90 degrees or approximately 90 but is not limited to these values. Once the position of the second vertical shaft 75 is fixed in the movement trajectory T1, the angle of the touch-panel screen 51 of the monitor 50 can be adjusted within the movement trajectory T2 so that the touch-panel screen 51 is positioned parallel to the back surface of the cover body 30 or tilted relative to the corner portion of the cover body 30.

The link mechanism 70 is configured to move the monitor position of the monitor 50 within a movement range in the X-axis direction at an upper height position on the back surface of the cover body 30, among the right-corner inclined position P3, the back-surface right-side position P4, and the back-surface center position P5, as shown in FIG. 10. When the examiner sets the touch-panel screen 51 of the monitor 50 at the back-surface center position P5 at an upper height position on the back surface of the cover body 30, it is positioned as shown with the solid lines in FIGS. 3 and 4. In this case, the examiner seated at the back side of the cover body 30 is positioned closer to the monitor 50 and the control lever 60, making it suitable for the touch-panel operation and the lever operation from the back side. The touch-panel screen 51 of the monitor 50 can also be set at the right-corner inclined position P3 at an upper height position on the back surface of the cover body 30. In this case, the examiner seated obliquely, facing the right corner of the cover body 30, is positioned closer to the monitor 50 and the control lever 60, making it suitable for the touch-panel operation and the lever operation from an oblique position near the corner of the cover body 30.

The advantageous effects of the ophthalmologic apparatus will be described.
(1) An ophthalmologic apparatus A includes a measurement portion 20 that is configured to measure a subject eye E; a cover body 30 that is configured to cover the measurement portion 20; a monitor 50 that is disposed outside of the cover body 30 and is configured to present a touch-panel screen 51 at a monitor position of the monitor 50; and a moving mechanism that is configured to move the monitor position of the monitor 50. The moving mechanism is a link mechanism 70 including at least first and second vertical shafts 73, 75 and at least first and second connecting arms 74, 76. The first and second connecting arms 74, 76 are rotatably connected by the first and second vertical shafts 73, 75 as a joint therebetween. The link mechanism 70 is configured to move the monitor position of the monitor 50 within a predetermined movement range between a front-side position and a back-side position in a front-back axis (Z-axis) direction of the ophthalmologic apparatus A at an upper height position on a side surface of the cover body 30. This invention enables selective setting of the monitor position to correspond to the selection of the operation target when the examiner operates from the side, while maintaining an economically advantageous moving mechanism. As a result, it allows switching between the lever operation and the touch-panel operation from the side. The link mechanism 70 has the flexibility to accommodate both the lever operation and the touch-panel operation, allowing the same link mechanism 70 to be commonly used for both the lever-equipped ophthalmologic apparatuses and the lever-free ophthalmologic apparatuses.
(2) The link mechanism 70 is provided on an upper position of the measurement portion 20. This invention ensures a high installation strength of the link mechanism 70 by installing the link mechanism 70 for moving the monitor 50 on the measurement portion 20, rather than on the cover body 30.
(3) The link mechanism 70 further includes a fixation plate 71 that is fixed to a side surface position of the measurement portion 20; and a support plate 72 that is connected to the fixation plate 71 and is placed on an upper surface position of the measurement portion 20. This invention can ensure the support strength of the link mechanism 70 with less deflection or shaking of the monitor 50 by this two-side support using the fixation plate 71 and the support plate 72, when the link mechanism 70 for moving the monitor 50 is supported on the measurement portion 20, as compared with the supporting on only the side surface or top surface of the measurement portion 20.
(4) The at least first and second vertical shafts 73, 75 include a first vertical shaft 73 and a second vertical shaft 75. The at least first and second connecting arms 74, 76 include a first connecting arm (first bent connecting arm) 74 and a second connecting arm 76. The first vertical shaft 73 is provided at an end position of the support plate 72. The first connecting arm 74 is connected to a first end of the first connecting arm 74 and the second vertical shaft 75 is connected to a second end of the first connecting arm 74. The second vertical shaft 75 is provided at a first end of the second connecting arm 76. The link mechanism 70 further includes a monitor attachment portion 77 that is connected to a second end of the second connecting arm 76. A back side of the monitor 50 is attached to the monitor attachment portion 77. This invention enables setting the monitor position within the movement range in the front-back direction (Z-axis direction) between the front-side position and the back-side position at least at the upper height position on the side surface of the cover body 30, while using the link mechanism 70 with a reduced number of components.
(5) In a plan view of the cover body 30, the first vertical shaft 73 is disposed at a position offset in a left-right axis (X-axis) direction from a center line CL at a rear position thereof. The center line CL extending in the front-back axis (Z-axis) direction. The first connecting arm 74 is a first bent connecting arm 74 including a radial arm portion 74a and a circumferential arm portion 74b and bent into an L-shape with the radial arm portion 74a and the circumferential arm portion 74b. The first bent connecting arm 74 is bent such that the circumferential arm portion 74b extends in a direction bringing the second vertical shaft 75 closer to the center line CL when the first bent connecting arm 74 rotates. This invention can set the monitor position in the movement range in the left-right axis direction (X-axis direction) including the center part of the upper position of the back surface of the cover body 30 when the first vertical shaft 73 is disposed at the offset position L0 offset from the center line CL. Additionally, this invention can set the monitor position by the inclined disposition at the upper position of the corner portion between the back surface and the side surface on the offset side of the first vertical shaft 73.

### Second Embodiment

The ophthalmologic apparatus A' according to the second embodiment is similar in configuration to the ophthalmologic apparatus A in the first embodiment. Accordingly, the following description will focus only on differences from the first embodiment, and the same or similar components are denoted by the same or similar reference signs as in the first embodiment. The ophthalmologic apparatus A' is an example of the ophthalmologic apparatus of the present invention and is characterized in that the first vertical shaft 73' is positioned on the center line CL by using an extended support plate 72', and the first connecting arm is configured as a first straight connecting arm 74', which directly connects the first vertical shaft 73' and the second vertical shaft 75 in a straight line.

The detailed configuration of the link mechanism 70' is described with reference to FIG. 11. The link mechanism 70' includes a semicircular movement range for moving the monitor position of the monitor 50. This movement range extends between a first side surface position and a second side surface position via the back surface position, at an upper height position on both side surfaces and the back surface. The link mechanism 70' includes two vertical shafts, two arms, a fixation plate 71, a support plate 72', a first vertical shaft 73', a first straight connecting arm (first connecting arm) 74', a second vertical shaft 75, a second connecting arm 76, and a monitor attachment portion 77.

Similar to the first embodiment, the support plate 72' is integrally connected with the fixation plate 71. However, the support plate 72' is longer than the support plate 72 in the first embodiment to allow the first vertical shaft 73 to be set on the center line CL.

As shown in FIG. 11, in a plan view of the cover body 30, the first vertical shaft 73' is positioned at a rear location on the center line CL extending in the Z-axis direction.

The first straight connecting arm 74' is an arm member that extends straight between the first and second vertical shafts 73', 75. The first straight connecting arm 74' has an arm length L3 extending between the first vertical shaft 73' and the second vertical shaft 75, allowing the monitor 50 to pass over both rear corner portions of the cover body 30, each of which is formed between a side surface and the back surface of the cover body 30. The other configurations of the link mechanism 70' are similar to those of the first embodiment, so their explanations are omitted.

The monitor position setting operation around the cover body is described with reference to FIG. 11. The link mechanism 70' is configured to move the monitor position of the monitor 50 within an arcuate movement range extending between the first side surface position and the second side surface position via the back surface position at the upper height position on both side surfaces and the back surface of the cover body 30. In other words, the second vertical shaft 75 of the link mechanism 70' follows a movement trajectory T3 within a predetermined arcuate angle range with the first vertical shaft 73' as its center and a radius equal to the arm length L3 of the first straight connecting arm 74'. For example, the predetermined arcuate angle range may be 270 degrees or approximately 270 degrees but is not limited thereto. As shown in FIG. 11, the arcuate movement range of the movement trajectory T3 includes a movement range of the monitor 50 in the Z-axis direction between the front side position P1 and the back side position P2 on the right-side surface. Furthermore, the arcuate movement range of the movement trajectory T3 also includes the movement range of the monitor 50 in the Z-axis direction between the back side position P8 and the front side position P9 on the left-side surface.

When the examiner sets the touch-panel screen 51 of the monitor 50 at the back side position P2 on the upper part of the right-side surface of the cover body 30 as shown with the solid lines in FIGS. 1 and 2, the position of the examiner becomes closer to the control lever 60, making it suitable for the lever operation from the right-side. In contrast, when the examiner sets the touch-panel screen 51 of the monitor 50 at the front side position P1 on the upper part of the right-side surface of the cover body 30, the position of the examiner becomes closer to the examinee, making it suitable for the touch-panel operation from the right-side.

When the examiner sets the touch-panel screen 51 of the monitor 50 at the back side position P8 on the upper part of the left-side surface of the cover body 30, the position of the examiner becomes closer to the control lever 60, making it suitable for the lever operation from the left-side. In contrast, when the examiner sets the touch-panel screen 51 of the monitor 50 at the front side position P9 on the upper part of the left-side surface of the cover body 30, the position of the examiner becomes closer to the examinee, making it suitable for the touch-panel operation from the left-side position.

As shown in FIG. 11, the arcuate movement range of the link mechanism 70' includes the movement range of the monitor 50 in the X-axis direction, extending between the right-corner inclined position P3 and the left-corner inclined position P7 via the back-surface's right-side position P4, the back-surface's center position P5, and the back-surface's left-side position P6.

When the examiner sets the touch-panel screen 51 of the monitor 50 at the back-surface's center position P5 on the upper part of the back surface of the cover body 30, it is suitable for the touch-panel operation and the lever operation by the examiner seated at the back center position. When the examiner sets the touch-panel screen 51 of the monitor 50 at the back-surface's right-side position P4 or the back-surface's left-side position P6 on the upper part of the back surface of the cover body 30, it is suitable for the touch-panel operation and the lever operation by the examiner seated at the back-surface's right-side or left-side position. When the examiner sets the touch-panel screen 51 of the monitor 50 at the right-corner inclined position P3 or the left-corner inclined position P7 on the upper part of the back surface of the cover body 30, it is suitable for the touch-panel operation and the lever operation by the examiner seated obliquely facing the right or left corner of the cover body 30.

The ophthalmologic apparatus A' according to the second embodiment provides the following advantageous effects in addition to any of the advantageous effects described in (1) to (5) for the ophthalmologic apparatus according to the first embodiment.

(6) **In** a plan view of the cover body 30, the first vertical shaft 73' is disposed on a center line CL at a rear position thereof. The center line CL extends in the front-back axis direction. The first connecting arm is a first straight connecting arm 74' that connects the first and second vertical shafts 73', 75 in a straight line. The first straight connecting arm 74' has an arm length L3 that allows the monitor 50 to pass over both rear corner portions of the cover body 30, each of rear corner portions defined between corresponding one of left and right side surfaces of the cover body 30 and a back surface thereof. This invention allows an even greater degree of freedom in setting the monitor position of the monitor 50 using the link mechanism 70', compared to the link mechanism 70 in the first embodiment when there is no restriction on the position for disposing the first vertical shaft 73'. In other words, when the first vertical shaft 73' is disposed on the center line CL, the monitor position can be set within the movement range in the Z-axis direction at the upper position of both the left and right side surfaces of the cover body 30. Furthermore, when the first vertical shaft 73' is disposed on the center line CL, the monitor position can also be set within the movement range in the X-axis direction at the upper position of the back surface of the cover body 30, as well as in the inclined disposition at the upper positions of both left and right corner portions.

### Third Embodiment

Now, the third embodiment will be described with reference to FIGS. 12 and 13. The following description will focus only on differences from the first and second embodiments and the same or similar components are denoted by the same or similar reference signs as in the first embodiment. The third embodiment is an example in which a monitor tilting mechanism 80 and a monitor sliding mechanism 90 are added between the monitor back surface of the monitor 50 and the monitor attachment portion 77 of the link mechanism 70 of the ophthalmologic apparatus A in the first embodiment or of the link mechanism 70' of the ophthalmologic apparatus A' in the second embodiment.

The configurations of the monitor tilting mechanism and the monitor sliding mechanism will be described. The link mechanism 70 or 70' includes, at its end portion, the monitor attachment portion 77 to which the monitor 50 is attached. The monitor tilting mechanism 80 is configured to rotate the monitor 50 about a horizontal shaft 81 when the monitor 50 is attached to the monitor attachment portion 77. The monitor sliding mechanism 90 is configured to slide the monitor 50 in upward and downward directions, while the monitor 50 is attached to the monitor attachment portion 77.

As shown in FIG. 12, the monitor 50 includes a monitor attachment plate 52, a first backside plate 54, and a second backside plate 55 on a monitor backside 53, as both the monitor tilting mechanism 80 and the monitor sliding mechanism 90 are provided. The first backside plate 54 is fixed in parallel with the monitor attachment plate 52.

The monitor tilting mechanism 80 is configured to rotate the second backside plate 55 relative to the first backside plate 54 about the horizontal shaft 81 in the direction of arrow R in FIG. 12. The horizontal shaft 81 is mounted on the upper end portions of both the first and second backside plates 54, 55.

The monitor sliding mechanism 90 is installed between the monitor backside 53 and the second backside plate 55. The monitor sliding mechanism 90 is configured to slide the monitor 50 vertically relative to the second backside plate 55, as shown with arrow S in FIG. 12. As shown in the left-side figure (a) of FIG. 13, the monitor sliding mechanism 90 may be configured as a parallel link-type monitor sliding mechanism 90', in which four links are connected together by four pins, with one of the upper and lower pins mounted on the monitor backside 53 and the other of them mounted on the second backside plate 55. Alternatively, as shown in the right-side figure (b) of FIG. 13, the monitor sliding mechanism 90 may be configured as a guide rail-type monitor sliding mechanism 90'', in which two sliders are respectively engaged with rail grooves of two sliding rails, with the sliding rails mounted on the second backside plate 55, and the sliders mounted on the monitor backside 53. The monitor sliding mechanism 90 is not limited to the configurations shown in the left-side figure (a) and the right-side figure (b) of FIG. 13, as long as it enables the monitor 50 to slide vertically.

The ophthalmologic apparatus according to the third embodiment provides the following advantageous effects in addition to any of the advantageous effects described in (1) to (5) for the ophthalmologic apparatus A in the first embodiment and in (6) for the ophthalmologic apparatus A' in the second embodiment.

(7) The ophthalmologic apparatus includes a monitor tilting mechanism 80 that is configured to rotate the monitor 50 about a horizontal shaft 81 when the monitor 50 is attached to the link mechanism 70, 70'. This invention enables setting the touch-panel screen 51 of the monitor 50 at an optimal angle for easy viewing, regardless of the examiner's standing or seated posture and height differences.

(8) The ophthalmologic apparatus includes a monitor sliding mechanism 90 that is configured to slide the monitor 50 in upward and downward directions when the monitor 50 is attached to the link mechanism 70, 70'. This invention allows the adjustment of the touch-panel screen 51 of the monitor 50 to match various eye position heights of different examiners when they are seated on a chair for the examination or measurement. Additionally, this invention enables the examiner to remain visible to the examinee by sliding the monitor 50 downward when the monitor 50 obstructs the examinee's view of the examiner.

The ophthalmologic apparatus has been described with reference to the first to third embodiments together with the drawings. However, the specific configurations of the ophthalmologic apparatus are not limited to those in the first to third embodiments. Design changes, additions, and modifications are permitted as long as they do not deviate from the gist of the inventions set forth in the attached claims.

Each of the link mechanisms 70, 70' in the first and second embodiments includes two vertical shafts and two arms, but it is not limited thereto. For example, the link mechanism may include three vertical shafts and three arms. Alternatively, it may include four or more vertical shafts and four or more arms. As the number of vertical shafts and arms increases, the link mechanism provides greater freedom in setting the monitor position, allowing for a more flexible positioning of the monitor.

Each of the link mechanisms 70, 70' in the first and second embodiments is installed at the upper position of the measurement portion 20, but it is not limited thereto. Alternatively, the link mechanism may be installed on the upper surface position of the cover body.

According to the first embodiment, in a plan view of the cover body 30, the first vertical shaft 73 is disposed at a position that is offset rightward along the X-axis (left-right axis) direction from the center line CL extending in the Z-axis direction at the rear position thereof. According to the second embodiment, in a plan view of the cover body 30, the first vertical shaft 73' is disposed at the rear position on the center line CL extending in the Z-axis direction. However, the first vertical shaft is not limited to the configurations in the first and second embodiments, where it is positioned either offset rightward from the center line CL or on the center line CL. For example, the first vertical shaft may be disposed at a position that is offset leftward from the center line CL, depending on the arrangement and shape of the measurement portion or other components.

The third embodiment shows an example in which both the monitor tilting mechanism 80 and the monitor sliding mechanism 90 are installed between the monitor attachment portion 77 and the monitor 50, but this configuration is not limited thereto. For example, neither the monitor tilting mechanism nor the monitor sliding mechanism may be installed, as in the first and second embodiments. Alternatively, only one of them may be installed between the monitor attachment portion and the monitor.

The ophthalmologic apparatus in the first and second embodiments is configured to observe, capture, and record ocular fundus images of the subject eyes and provide them as electronic images for diagnosis. However, the ophthalmologic apparatus is not limited to the configurations in the first and second embodiments. It may also be configured to observe, capture, and record ocular fundus tomographic images and anterior ocular segment images, in addition to ocular fundus images, of the subject eyes, and to provide them as electronic images for diagnosis. Furthermore, the ophthalmologic apparatus can be applied to various types of apparatuses, as long as it includes a monitor and a moving mechanism that moves the monitor position to present a touch-panel screen.

The present invention further discloses the following.
(1) An ophthalmologic apparatus comprising:
   a measurement portion that is configured to measure a subject eye;
   a cover body that is configured to cover the measurement portion;
   a monitor that is disposed outside of the cover body and is configured to present a touch-panel screen at a monitor position of the monitor; and
   a moving mechanism that is configured to move the monitor position of the monitor,
   wherein the moving mechanism is a link mechanism comprising:
      at least first and second vertical shafts and
      at least first and second connecting arms, the first and second connecting arms being rotatably connected by the first and second vertical shafts as a joint therebetween, and
   wherein the link mechanism is configured to move the monitor position of the monitor within a predetermined movement range between a front-side position and a back-side position in a front-back axis direction of the ophthalmologic apparatus at an upper height position on a side surface of the cover body.
(2) The ophthalmologic apparatus according to the above (1), wherein the link mechanism is provided on an upper position of the measurement portion.
(3) The ophthalmologic apparatus according to the above (2), wherein the link mechanism further comprises:
   a fixation plate that is fixed to a side surface position of the measurement portion; and
   a support plate that is connected to the fixation plate and is placed on an upper surface position of the measurement portion.
(4) The ophthalmologic apparatus according to the above (3), wherein
   the at least first and second vertical shafts comprise a first vertical shaft and a second vertical shaft,
   the at least first and second connecting arms comprise a first connecting arm and a second connecting arm,
   the first vertical shaft is provided at an end position of the support plate,
   the first vertical shaft is connected to a first end of the first connecting arm and the second vertical shaft is connected to a second end of the first connecting arm,
   the second vertical shaft is provided at a first end of the second connecting arm, and
   wherein the link mechanism further comprises a monitor attachment portion that is connected to a second end of the second connecting arm, a back side of the monitor being attached to the monitor attachment portion.
(5) The ophthalmologic apparatus according to the above (4), wherein, in a plan view of the cover body, the first vertical shaft is disposed at a position offset in a left-right axis direction from a center line at a rear position thereof, the center line extending in the front-back axis direction,
   wherein the first connecting arm is a first bent connecting arm comprising a radial arm portion and a circumferential arm portion and bent into an L-shape, and
   wherein the first bent connecting arm is bent such that the circumferential arm portion extends in a direction bringing the second vertical shaft closer to the center line when the first bent connecting arm rotates.
(6) The ophthalmologic apparatus according to the above (4), wherein, in a plan view of the cover body, the first vertical shaft is disposed on a center line at a rear position thereof, the center line extending in the front-back axis direction,
   wherein the first connecting arm is a first straight connecting arm that connects the first and second vertical shafts in a straight line, and
   wherein the first straight connecting arm has an arm length that allows the monitor to pass over both rear corner portions of the cover body, each of rear corner portions defined between corresponding one of left and right side surfaces of the cover body and a back surface thereof.
(7) The ophthalmologic apparatus according to any one of the above (1) to (6), further comprising a monitor tilting mechanism that is configured to rotate the monitor about a horizontal shaft when the monitor is attached to the link mechanism.
(8) The ophthalmologic apparatus according to any one of the above (1) to (7), further comprising a monitor sliding mechanism that is configured to slide the monitor in upward and downward directions when the monitor is attached to the link mechanism.

### List of Reference Signs

- A, A': ophthalmologic apparatus
- 10: apparatus pedestal
- 20: measurement portion
- 30: cover body
- 40: face support
- 50: monitor
- 60: control lever
- 70: link mechanism (moving mechanism)
- 80: monitor tilting mechanism
- 90: monitor sliding mechanism
- E: subject eye

## Claims

1. An ophthalmologic apparatus (A, A') comprising:
a measurement portion (20) that is configured to measure a subject eye (E);
a cover body (30) that is configured to cover the measurement portion (20);
a monitor (50) that is disposed outside of the cover body (30) and is configured to present a touch-panel screen (51) at a monitor position of the monitor (50); and
a moving mechanism that is configured to move the monitor position of the monitor (50),
wherein the moving mechanism is a link mechanism (70, 70') comprising:
at least first and second vertical shafts (73, 73', 75) and
at least first and second connecting arms (74, 74', 76), the first and second connecting arms (74, 74', 76) being rotatably connected by the first and second vertical shafts (73, 73', 75) as a joint therebetween, and
wherein the link mechanism (70, 70') is configured to move the monitor position of the monitor (50) within a predetermined movement range between a front-side position and a back-side position in a front-back axis direction of the ophthalmologic apparatus (A, A') at an upper height position on a side surface of the cover body (30).

2. The ophthalmologic apparatus (A, A') according to claim 1, wherein the link mechanism (70, 70') is provided on an upper position of the measurement portion (20).

3. The ophthalmologic apparatus (A, A') according to claim 2, wherein the link mechanism (70, 70') further comprises:
a fixation plate (71) that is fixed to a side surface position of the measurement portion (20); and
a support plate (72, 72') that is connected to the fixation plate (71) and is placed on an upper surface position of the measurement portion (20).

4. The ophthalmologic apparatus (A) according to claim 3, wherein
the at least first and second vertical shafts (73, 75) comprise a first vertical shaft (73) and a second vertical shaft (75),
the at least first and second connecting arms (74, 76) comprise a first connecting arm (74) and a second connecting arm (76),
the first vertical shaft (73) is provided at an end position of the support plate (72),
the first vertical shaft (73) is connected to a first end of the first connecting arm (74) and the second vertical shaft (75) is connected to a second end of the first connecting arm (74),
the second vertical shaft (75) is provided at a first end of the second connecting arm (76), and
wherein the link mechanism (70) further comprises a monitor attachment portion (77) that is connected to a second end of the second connecting arm (76), a back side of the monitor (50) being attached to the monitor attachment portion (77).

5. The ophthalmologic apparatus (A) according to claim 4, wherein, in a plan view of the cover body (30), the first vertical shaft (73) is disposed at a position offset in a left-right (X) axis direction from a center line (CL) at a rear position thereof, the center line (CL) extending in the front-back (Z) axis direction,
wherein the first connecting arm (74) is a first bent connecting arm (74) comprising a radial arm portion (74a) and a circumferential arm portion (74b) and bent into an L-shape, and
wherein the first bent connecting arm (74) is bent such that the circumferential arm portion (74b) extends in a direction bringing the second vertical shaft (75) closer to the center line (CL) when the first bent connecting arm (74) rotates.

6. The ophthalmologic apparatus (A') according to claim 4, wherein, in a plan view of the cover body (30), the first vertical shaft (73') is disposed on a center line (CL) at a rear position thereof, the center line (CL) extending in the front-back (Z) axis direction,
wherein the first connecting arm (74') is a first straight connecting arm (74') that connects the first and second vertical shafts (73', 75) in a straight line, and
wherein the first straight connecting arm (74') has an arm length (L3) that allows the monitor (50) to pass over both rear corner portions of the cover body (30), each of rear corner portions defined between corresponding one of left and right side surfaces of the cover body (30) and a back surface thereof.

7. The ophthalmologic apparatus (A, A') according to any one of claims 1 to 6, further comprising a monitor tilting mechanism (80) that is configured to rotate the monitor (50) about a horizontal shaft (81) when the monitor (50) is attached to the link mechanism (70, 70').

8. The ophthalmologic apparatus (A, A') according to any one of claims 1 to 7, further comprising a monitor sliding mechanism (90) that is configured to slide the monitor (50) in upward and downward directions when the monitor (50) is attached to the link mechanism (70, 70').
